(19) **Europäisches Patentamt**

**European Patent Office**

**Office européen des brevets**

(11) Veröffentlichungsnummer: **0 120 376**
**B1**

(12) **EUROPÄISCHE PATENTSCHRIFT**

(45) Veröffentlichungstag der Patentschrift: 24.10.90

(51) Int. Cl.⁵: **C 12 Q 1/68** // C07H21/00

(21) Anmeldenummer: 84102594.3

(22) Anmeldetag: 09.03.84

(54) **Verfahren zur Durchführung von Hybridisierungsreaktionen.**

(30) Priorität: 22.03.83 DE 3310337

(43) Veröffentlichungstag der Anmeldung:
03.10.84 Patentblatt 84/40

(45) Bekanntmachung des Hinweises auf die
Patenterteilung:
24.10.90 Patentblatt 90/43

(84) Benannte Vertragsstaaten:
DE FR GB SE

(56) Entgegenhaltungen:
GB-A-2 019 408

CHEMICAL ABSTRACTS, Band 96, Nr. 5, 1.
Februar 1982, Seite 233, Nr. 30348u, Columbus,
Ohio, USA; N.P. KISELEVA et al.:
"Formaldehyde-induced tight binding of protein
to RNA in particles of a rod-like virus. Nature
and specificity of the crosslinks" & BIOKHIMIYA
(MOSCOW) 1981, 46(11), 2019-2023

(73) Patentinhaber: EUROPÄISCHES
LABORATORIUM FÜR MOLEKULARBIOLOGIE
(EMBL)
Meyerhofstrasse 1
D-6900 Heidelberg 1 (DE)

(72) Erfinder: Renz, Manfred, Dr.
Panoramastrasse 145
D-6900 Heidelberg 1 (DE)

(74) Vertreter: Pennant, Pyers et al
Stevens, Hewlett & Perkins 5 Quality Court
Chancery Lane
London, WC2A 1HZ (GB)

(56) Entgegenhaltungen:
CHEMICAL ABSTRACTS, Band 97, Nr. 5, 4.
August 1982, Seite 200, Nr. 34867f, Columbus,
Ohio, USA; C. VAN EEKELEN et al.: "Detection
of a cellular polypeptide associated with
adenovirus-coded VA RNA using in vitro
labeling of proteins cross-linked in RNA" &
NUCLEIC ACIDS RES. 1982, 10(10), 3039-3052

(56) Entgegenhaltungen:
CHEMICAL ABSTRACTS, Band 71, Nr. 5, 4.
August 1969, Seite 16, Nr. 18846f, Columbus,
Ohio, USA; N.K. DAS et al.: "Binding of labeled
ribonucleic acid to basic proteins, a major
difficulty in ribonucleic acid-deoxyribonucleic
acid hybridization in fixed cells in situ"

## Beschreibung

Die Erfindung betrifft ein Verfahren zur Durchführung von Hybridisierungsreaktionen und ein für dieses Verfahren geeignetes Nachweismittel.

Die Hybridisierung ist ein Mittel zum Feststellen der Identität von Basensequenzen bei zwei verschiedenen Polynukleotidketten. Zwei DNA-Ketten mit komplementären Basensequenzen bilden eine Doppelstrangstruktur miteinander bei der Abkühlung in Lösung und genauso verhalten sich eine DNA-Kette und eine RNA-Kette mit komplementärer Struktur unter Bildung eines DNA-RNA-Hybrids. Durch Hybridisierung lassen sich daher zahlreiche Probleme der Nukleotidchemie lösen, beispielsweise die Auffindung und Isolierung bestimmter Gene oder Genbruchstücke, die Kartierung von Gensequenzen und dergleichen. Insbesondere im Rahmen der Gentechnologie spielt die Auffindung bestimmter gesuchter Gene eine entscheidende Rolle und Hybridisierung ermöglicht dies.

Zur Durchführung der Hybridisierungsreaktion verwendet man zweckmäßig eine Nukleinsäurekette mit der zur gesuchten Nukleinsäure komplementären Struktur, welche in geeigneter Weise für die Wiederauffindung markiert ist. Üblicherweise erfolgt diese Markierung bisher durch enzymatische Einführung von Radioisotopen-tragenden Nukleotidtriphosphaten in die für die Suche verwendete komplementäre Nukleinsäure. Ein erheblicher Nachteil dieses Verfahrens besteht in der Notwendigkeit, mit radioaktiven Substanzen zu arbeiten, was sowohl umfangreiche Sicherheitsvorkehrungen erfordert als auch aufwendige Apparaturen voraussetzt.

Man hat daher bereits Versuche unternommen, die radioaktive Markierung durch andere Markierungen zu ersetzen.

So wurden neuerdings Nukleotidanaloge synthetisiert, die kovalent verknüpftes Biotin an einem Pyrimidin- oder Purinring enthalten. Diese Nukleotidderivate sind Substrate für RNS- und DNS-Polymerasen und die unter der Einwirkung dieser Enzyme gebildeten Polynukleotide hybridisieren spezifisch mit ihren Komplementärsequenzen. In Verbindung mit einem Affinitäts- (z. B. Avidin-Peroxidase) oder einem immunologischen (z. B. Biotin-Antikörper in Verbindung mit einem zweiten Peroxidase tragenden Antikörper) Nachweissystem können diese Proben als Alternative zu radioaktiv markierten Nukleinsäuren verwendet werden. Auch wurden schon Biotinreste mit RNS über Cytochrom C verknüpft und die Hybridisierungen elektronenmikroskopisch mit dem Avidin-Nachweissystem lokalisiert (Chromosoma/Berl. 53, 107—117 (1975)).

Dieses Verfahren ist jedoch immer noch relativ aufwendig, da es mehrere Syntheseschritte erfordert. Es besteht daher unverändert ein Bedarf nach einem einfacheren Hybridisierungsverfahren und einem Hybridisierungsreagenz, welches sich als Probe eignet und einfach hergestellt werden kann und die Empfindlichkeit der Hybridisierungstechnik erhöht.

Aufgabe der Erfindung ist es daher, ein derartiges Verfahren und Mittel zu schaffen.

GB 82109408 betrifft Nachweistechniken, die das Hybridisieren einer Nukleinsäuresequenz mit einer markierten Probe einschließen. Die Markierung umfaßt ein Enzym, das jedoch mit der Probe erst nach der Hybridisierung verbunden wird.

In Chemical Abstract, 1982, 96: 30348 wird engas, Formaldehyd-induziertes Verbinden von Protein mit RNS in Teilchen eines stäbchenförmigen Virus beschrieben.

Ein Verfahren gemäß der vorliegenden Erfindung zum Nachweist einer Target-Polynukleotid-Sequenz in Polynukleotid-Material durch In-Kontakt-Bringen des Materials unter Hybridisierungsbedingungen mit einer Probe, enthaltend eine Polynukleotid-Sequenz, die komplementär zu der Target-Sequenz ist, und durch anschließendes Verwenden der Probe zum Nachweis der Target-Sequenz in dem Material, ist zum einen dadurch gekennzeichnet, daß die Probe durch Reaktion in Lösung der Einzelstrang-Komplementär-Polynukleotid-Sequenz mit einem nachweisbaren positive geladenen Nukleinsäure-bindenden Protein oder Polyamin und kovalentes Verbinden des resultierenden Komplexes mit einem Vernetzungsmittel gebildet wurde. Unter einem anderen Aspekt ist das Verfahren dadurch gekennzeichnet, daß die Probe durch Reaktion in Lösung der Einzelstrang-Komplementär-Polynukleotid-Sequenz mit einem nachweisbaren Protein, das mit einem positiv geladenen Protein oder Polyamin modifiziert wurde und kovalentes Verbinden der resultierenden Komplexes mit einem Vernetzungsmittel gebildet wurde. Das Polyamin kann ein Polyethylenimin sein.

Unter einem anderen Aspekt liefert die Erfindung das Reaktionsprodukt eines Enzyms mit einem Polyethylenimin, wobei das Reaktionsprodukt die Fähigkeit besitzt kovalent mit einem Vernetzungsmittel an eine Polynukleotid-Sequenz verbunden zu werden.

Unter einem noch anderen Aspekt liefert die vorliegende Erfindung ein Verfahren zur Herstellung eines Enzym-Polyamin-Konjugats, wobei das Verfahren das kovalente Anbringen von Benzochinon-Molekülen an ein Enzym und das Reagieren des resultierenden Zwischenstoffes mit einem Polyamin umfaßt.

Als positiv geladene Proteine oder Polyamine werden im Rahmen der Erfindung solche verstanden, die entweder diese Eigenschaft an sich besitzen oder, falls sie negativ geladen oder neutral sind, mit einem entsprechend positiv geladenen Protein oder Polyamin modifiziert wurden. Hieraus ergibt sich auch die überraschend aufgefundene Tatsache, daß sich jedes beliebige Protein in ein DNA-bindendes Protein überführen läßt, indem man es mit einem positiv geladenen Protein oder Polyamin modifiziert.

Die Erfindung liefert somit ein einfaches Verfahren zur Erzeugung von Hydridisierungsproben, das es erlaubt, auch große Proteinmoleküle als Markierungen einzuführen. Das Verfahren baut auf der Tatsache auf, daß ein positiv geladenes Proten oder Polyamin durch elektrostatische Wechselwirkungen an eine Nukleinsäure bei geringer Ionenstärke bindet und somit Vernetzungsreaktionen zwischen Nukleinsäure und Protein oder Polyamin begünstigt. Vorzugsweise wird einzelsträngige DNS und ein Lysin-reiches DNS-bindendes chromosomales Protein, wie z. B. Histon H1, benutzt. Der hohe Lysin-Gehalt derartiger Protein erfüllt zwei Anforderungen:

1. Es wird eine sehr enge Bindung zu Nukleinsäuren erhalten und daher eine sehr wirksame Vernetzung mit geringen Konzentrationen an Vernetzungsmittel wie z. B. Glutaraldehyd innerhalb kurzer Reaktionszeiten ermöglicht. So hergestellte Protein-Nukleinsäure-Verbindungen (Proben) sind nicht einzelsträngige DNS markiert mit Protein, sondern stellen kovalent verbundene Einzelstrang-DNS-Protein-Einheiten dar mit einem Protein: DNS-Massenverhältnis von nahezu 1. Nach Hydridisierung kann man jetzt die Komplementärsequenzen nachweisen durch Suche nach dem Protein (z. B. mit einem Antikörper).

2. Proteine oder Polyamine können chemisch einfach modifiziert werden. Im Falle des bevorzugten Histon H1 erfolgt die Modifizierung z. B. an der -Aminogruppe der Lysinreste. Dabei können Markierungsgruppen oder -verbindungen direkt oder über Brückenbildnerverbildungen wie z. B. cyclische N-Succinimidester an die Aminogruppe fixiert werden. Eine Markierung der Lysin-Aminogruppe hat wenig Einfluß auf die Nukleinsäurebindende Eigenschaft des Proteins, da nicht alle Lysinreste für starke Nukleinsäure-Proteinwechselwirkungen benötigt werden. Mit dieser Methode kann man viele Histon H1-Moleküle (jetzt markiert) mit Einzelstrang-DNS vernetzen und Marker mit einer großen Zahl von Markierungen erhalten, die nach Hybridisierung mit einem Affinitäts- oder immunologischen Nachweissystem leicht zu bestimmen sind. In Blot Hybridisierungsexperimenten kann gezeigt werden, daß unter identischen Reaktionsbedingungen markierte Proben entsprechend der erfindungsgemäßen Methode mit ihren Komplementär-Sequenzen mit hoher Spezifität reagieren und hierin nicht unterscheidbar sind von Nick-translatierten oder endmarkierten Proben.

Als positiv geladene, mit Nukleinsäuren-bindenden Protein eignen sich im Rahmen der Erfindung prinzipiell alle positiv geladenen Polymeren und Oligomeren von Aminosäuren, natürlichen oder synthetischen Ursprungs, wie beispielsweise Polylysin, chromosomale Proteine einschließlich der Histone, einzelstrangspezifisch bindende Proteine wie z. B. Gen-32-Protein. Ebenfalls geeignet sind Polyamine. Bevorzugt werden unter diesen die natürlich vorkommenden sogenannten "Polyamine" wie Spermidin, Spearmin, Putrescin und dergleichen, die teilweise eigentlich Oligoamine darstellen. Geeignet sind aber auch synthetische Polyamine.

Das einzelsträngige Polynukleotid kann ebenfalls natürlichen oder synthetischen Ursprungs sein, vorzugsweise handelt es sich um eine DNS oder RNS. Als Hybridisierungsreaktionspartner kommen ebenfalls DNS und RNS in Frage, so daß die Hybridisierungsreaktion selbst DNS mit DNS, DNS mit RNS und RNS mit RNS betreffen kann.

Die Vernetzung des gebildeten Komplexes aus einzelsträngigem Polynukleotid und dem positiv geladenen Nukleinsäure bindenden Protein oder Polyamin erfolgt mit üblichen Vernetzungsmitteln, welche die funktionellen Gruppen des Proteins mit den funktionellen Gruppen der Polynukleinsäure zu verbinden vermögen. Besonders geeignet sind difunktionelle Vernetzungsmittel wie Dialdehyde, beispielsweise Glutaraldehyd, Diepoxide und generell Verbindungen, welche zwei zur Alkylierung in wässriger Lösung geeignete funktionelle Gruppen tragen. Typische hierfür geeignete Gruppen sind aktivierte Carboxylgruppen wie Säurechloride, Säurebromide, Säureanhydride, durch benachbarte Doppelbindungen aktivierte Halogenatome und dergleichen.

Um das Hybridisierungsprodukt sichtbar zu machen, muß das darin enthaltene Protein bestimmbar sein, z. B. durch eine Markierung.

Die Markierung des Proteins bzw. Polyamins kann entweder schon vor Durchführung der Komplexbildungsreaktion erfolgen oder am bereits gebildeten und gegebenenfalls auch bereits vernetzten Komplex durchgeführt werden. Die Durchführung der Markierung erfolgt nach den hierfür bekannten Methoden der Proteinchemie, die dem Fachmann geläufig sind und hier nicht näher erläutert werden müssen. Als Markierungssystem kommt sowohl eine radioaktive Markierung als auch eine Markierung mit Farbstoffen, Farbstoffkomponenten oder biologisch aktiven Proteinen wie z. B. Enzymen in Betracht.

Anstelle einer Markierung des für die Komplexbildung verwendeten positiv geladenen Nukleinsäurebindenden Proteins oder Polyamins ist es auch möglich, das Protein oder Polyamin durch eine immunologische Reaktion nachzuweisen, wobei der Immunreaktionspartner in diesem Falle markiert sein muß oder mit einem zweiten Antikörper nachgewiesen wird. Die bestimmbaren Gruppen als solche können entweder direkt bestimmbar sein wie bei radio aktiven- oder Farbstoffmarkierungen, oder indirekt, z. B. aufgrund ihrer enzymatischen Aktivität, wie dies aus dem Enzym-Immuno-Assay-Verfahren bekannt ist.

Die Lösung für die Durchführung der Komplexbildungsreaktion zwischen Protein oder Polyamin und einzelsträngigem Polynukleotid soll eine geringe Ionenaktivität aufweisen. Vorzugsweise soll die Ionenstärke niedriger als 50 mM sein.

Die Hybridisierungsreaktion selbst erfolgt nach den hierfür bekannten Methoden durch einfaches Zusammengeben des erfindungsgemäß erhaltenen kovalent vernetzten Komplexes aus dem einzelsträngigen Polynukleotid und dem positiv geladenen Nukleinsäure-bindenden Protein oder Polyamin mit einer Lösung, welche die gesuchte komplementäre Nukleinsäure enthält und Inkubation, zweckmäßig bei etwas erhöhter Temperatur. Die Sichtbarmachung und Isolierung der gebildeten Hybride erfolgt ebenfalls nach dem Fachmann hierfür bekannten Methoden.

Unter einem anderen Gesichtspunkt liefert die Erfindung eine Probe, die zur Verwendung in Hybridisierungsreaktionen geeignet ist, dadurch gekennzeichnet, daß die Probe durch Reaktion in Lösung einer einzelsträngigen Polynukleotid-Sequenz, die komplementär zu einer gewünschten Target-Polynukleotid-Sequenz ist, entweder mit einem nachweisbaren, positiv geladenen Nukleinsäure-bindenden Protein oder Polyamin oder mit einem nachweisbaren Protein, das mit einem positive geladenen Protein oder Polyamin modifiziert wurde, und durch kovalentes Verbinden des resulierenden Komplexes mit einem Vernetzungsmittel gebildet wurde.

Vorzugsweise enthält das Reagenz als Proteinkomponente Histon H1. Ebenfalls bevorzugt ist ein Massenverhältnis von Protein und Nukleinsäure von etwa 1:1.

Durch die Erfindung wird ein Verfahren und ein Reagenz für die Durchführung von Hybridisierungsreaktionen zur Verfügung gestellt, welches als Probe eine chemisch hergestellte Verbindung verwendet, die mit hoher Ausbeute (mehr als 90%) anfällt, keine Polymerasen zu ihrer Herstellung benötigt und die Abtrennung von Vorläufern und Endprodukten überflüssig macht. Der Zeitaufwand ist sehr gering, die Polynukleotide für die Probenbereitung müssen nicht im hochreinen Zustand eingesetzt werden. Die dem Fachmann bekannten Standardmethoden für die Blot-Hybridisierung können unverändert benutz werden.

Die folgenden Beispiele erläutern das Verfahren der Erfindung unter Verwendung von markierten Polynukleotid-Histon, H1-Verbindungen als Proben für Hybridisierungsreaktionen.

Beispiel 1
A) Histon H1-Isolierung

Histon H1 (21 Kd) wurde durch Perchlorsäure-Extraktion aus Kälberthymuskernen isoliert wie bereits beschrieben in BBA Band 62, Seite 608 bis 609, 1962. Lyophilisiertes Histon H1 wurde in Wasser gelöst (5 mg/ml) und bei −20°C aufbewahrt.

B) Biotin-Markierung

Biotinyl-N-hydroxysuccinimidester (BHSE) wurde hergestellt wie beschrieben in Methods Enzymology, Band 62, Seite 308 bis 315, 1979, 250 mg Biotin (Merck), 250 µ Ci (1,4 µg) $^3$H-Biothin (NEN), 150 mg N-Hydroxysuccinimid (Merck) werden in 3 ml Dimethylformamid (DMF) gelöst und 200 mg Dicyclohexylcarbodiimid zugegeben. Die Mischung wird 16 Stunden bei 20°C gerührt. Der Niederschlag wird abfiltriert und das Filtrat im Vakuum getrocknet. Der Rückstand wird mit Äther gewaschen, aus Isopropanol umkristallisiert und so für die Biotinmarkierung eingesetzt.

Histon H1 wurde in verschiedener Weise mit steigenden Mengen an BHSE biotinyliert. Je 5 µg BHSE (gelöst in 10 µl DMF), 25 µg BHSE (in 10 µl DMF) und 250 µg (in 10 µl DMF) werden zu einer die gleiche Menge Histon H1 (je 1 mg in 300 µl 50 mM NaHCO$_3$) enthaltenen Lösung zugegeben und 1 Stunde bei 20°C inkubiert. Nach Dialyse gegen 5 mM Natriumphosphat (pH 6,8) werden die drei Proben bei −20°C aufbewahrt. Die Konzentrationen der Biotin markierten Histon H1-Lösungen werden durch Vergleich mit nicht modifiziertem Histon H1 durch SDS-Polyacrylamidgel-Elektrophorese bestimmt. Die Zahl der Biotinreste in den drei Präparationen wurde durch den in BHSE enthaltenen Tritium-Marker abgeschätzt und ergab 2,7 bzw. 20 pro Histon H1 Molekül.

C) Polynukleotid-Histon H1-Komplexbildung

Zirkuläre DNS (Plasmide) wie auch große lineare DNS-Moleküle (Phage-Lamda DNS) wurden mit Restriktionsendonukleasen linearisiert oder gespalten zu einer Durchschnittsgröße von ungefähr 4 kb. Im Verlauf der Untersuchung wurde festgestellt, daß kleinere DNS-Fragmente stärkere Hybridisierungssignale ergeben. Daher wurde die DNS mit Enzymen wie SAU 3 A oder Hae III gespalten. Die gespaltene DNS wurde ohne weitere Reinigung eingesetzt. 1 µg DNS (in nicht mehr als 20 µl Spaltpuffer) wurde mit 180 µl frisch bereitetem 5 mM Natriumphosphatpuffer (pH 6,8) verdünnt, durch Hitze denaturiert (100°C, 3 Minuten) und 3 Minuten im Eisbad abgekült. Zuerst wurde Histon H1 (zwischen 0,8 und 1,0 µg in ungefähr 5 µl Phosphatpuffer) zugegeben und danach 20 µl einer 2,5 %igen Glutaraldehydlösung. Die Probe wurde 10 Minuten bei 30°C inkubiert und direkt in das das Nitrocellulosepapier und die Hybridisierungslösung enthaltene Hybridisierungsgefäß gegeben.

D) Hybridisierung

Die zu untersuchende DNS wurde mit Restriktionsendonukleasen gespalten, durch Agarosegel-Elektrophorese fraktioniert und nach der Southern-Methode (J. Mol. Biol. 98, 503—517, 1975) auf Nitrocellulose-Papier übertragen. Die Papiere werden 1 Stunde bei 37°C in 10×Denhardts-Lösung (BBA, 23, 641 bis 645, 1966), 4×SET (1×SET=0,15 M NaCl, 0,03 M Tris×HCl, pH 8, 1 mM EDTA) eingeweicht und in Plastikwannen überführt, die zwischen 10 und 20 ml reiner Hybridisierungsmischung (50% deionisiertes Formamid, 2×Denhardts-Lösung, 4×SET, 0,1% SDS und 20 µg/ml Hefe T-RNS) enthalten, für eine Stunde bei 37°C inkubiert und dann nach Zugabe der Probe 16 bis 20 Stunden bei 37°C leicht geschwenkt.

Die Nitrocellulose-Papiere wurden dann 60 Minuten bei 37°C mit zwei Wechseln einer 50% Formamid, 0,2% SDS und 5×SSC (einmal SSC=0,15 M NaCl, 0,015 M Natriumcitrat) enthaltenen Lösung und 40 Minuten bei 20°C mit zwei Wechseln einer zweimal SSC enthaltenen Lösung gewaschen. Die Filter wurden getrocknet und mit einem Biotin-Nachweissystem entwickelt.

E) Visualisierung der Proben mit Avidin-Peroxidase

Die Nitrocellulosepapiere wurden zuerst 20 Minuten mit einer 10 µg/ml Poly-1-Lysin HBR, MW 220.000 (Sigma), 0,1 M Tris HCl (pH 7,5)

enthaltenen Lösung und dann mit einer 3% Rinderserumalbumin (BSA), 0,1 M Tris HCl (pH 7,4) enthaltenen Lösung 50 Minuten inkubiert und nachfolgend 60 Minuten lang mit der Avidin-Peroxidase-Lösung (1 M NaCl, 0,1 M Tris HCl, pH 7,4, 0,1% Triton X-100, 0,1% BSA und 1 µg/ml Avidin-Peroxidase, E. Y. Laboratories, California). Nach 20 Minuten Waschen mit zwei Wechseln einer 1 M NaCl, 0,1 M Tris HCl, pH 7,4, 0,1% BSA und 0,1% Triton X-100 enthaltenen Lösung, wurden die Papiere mit der Färbelösung (10 ml 0,1 M Tris HCl, pH 7,4, 2 ml Äthanol in den 6 mg 3,3′-Dianisidin gelöst wurden, 6 µl 30% $H_2O_2$) inkubiert. Die Farbentwicklung (braune Banden) findet in 5 bis 10 Minuten statt. Alle Inkubationsschritte wurden bei 20°C unter leichtem Schwenken ausgeführt.

Beispiel 2

Es wurde, wie in Beispiel 1 beschrieben gearbeitet, jedoch wurde anstelle einer Biotin-Markierung eine Markierung mit [125]J angewendet. Die Markierung von Histon H1 wurde wie folgt vorgenommen:

Bei 0°C wurden zu 1mCi festem Mono-jodo ([125]J)-Bolton-Hunter Reagenz (2000 Ci/mMol) (NEN) 60 µl einer zwischen 120 und 150 µg Histon H1 und 100 mM Natriumborat (pH 8,9) enthaltenen Lösung zugegeben und für eine Stunde stehen gelassen. Danach wurden 10 µl 1 M Glycin in 0,5 M Natriumborat zugegeben. Die Lösung wurde durch eine mit 50 µg Histon H1 konditionierten Säule (silanisierte Pasteurpipette) gelfiltriert (Sephadex G 100: Molekularsiebmat. auf Basis von vernetztem Dextran) in 5 mM Natriumphosphat, pH 6,8. [125]J-Histon H1 enthaltene Fraktionen wurden bei −20°C aufbewährt.

Beispiel 3

Für Genkartierung wurde ein Drosophila virilis Insert tragender λ-Clon mit verschiedenen Restriktionsendonukleasen gespalten, nach Größe getrennt, auf Nitrocellulosepapier übertragen und gleichzeitig mit einem erfindungsgemäßen Reagenz aus λ-DNS und [125]J-Histon H1 und einem erfindungsgemäßen Reagenz aus einem Plasmid (das Drosophila virilis Sequenzen des λ-Clones enthält) und Biotin-Histon H1 hybridisiert. Als Restriktionsendonukleasen wurden Eco R1, Bam H1, Hind III und Sal I verwendet. Durch Belichtung eines Röntgenfilmes und anschließende Entwicklung mit einem Biotin-Nachweise-System konnten die einzelnen Bruckstücke, die mit den verschiedenen Enzymen erhalten worden waren, identifiziert werden, welche jeweils die komplementäre Polynukleinsäure enthielten. Auf diese Weise war eine rasche Kartierung des Gens möglich.

Beispiel 4

20 mg (5×10[3] Einheiten) Meerrettichperoxidase (Grad 1 Boehringer Mannheim) wurden in 220 µl von 90 mM Natriumphosphat, pH 6,0 gelöst und dann mit 60 µ einer Lösung enthaltend 30 mg p-Benzochinon in 1 ml Ethanol versetzt. Die Mischung wurde eine Stunde bei 37°C im Dunklen reagieren gelassen. Peroxidase mit kovalent gebundenen Benzochinonmolekülen wurde von nicht umgesetztem Benzochinon durch Gelfiltration abgetrennt unter Verwendung einer 6 ml Säule Sephadex G 100 in 0,15 M NaCl ohne Puffer. Die braungefärbten Fraktionen (etwa 1,8 ml) wurden vereinigt und die Kupplungsreaktion in Gang gesetzt durch Anhebung des pH-Wertes durch Zusatz von 180 µl 1 M NaHCO$_3$ und Zusatz von 2,7 µl (133 µg) einer Polyethyleniminlösung (Polymin G 35 BASF). Die Reaktionsmischung wurde 14 Stunden bei 37°C im Dunkeln gehalten und dann gegen 5 mM Natriumphosphat, pH 6,8, dialysiert und bei 3°C gelagert (Lösung A). Die Proteinkonzentration der Lösung A betrug ca. 7 µg/µl. Während dreimonatiger Lagerung wurde kein Aktivitätsverlust beobachtet.

Größe und Zusammensetzung der Peroxidasekonjugate wurde durch SDS-Polyacrylamid Gelelektrophorese unter Verwendung von [125]-I-Polyethylenimin (markiert mit Bolton-Hunter-Reagenz) und durch Vergleich von Coomassie-blaugefärbten Gelen mit den entsprechenden Autoradiogrammen bestimmt. Dabei ergab sich, daß alle Peroxidasemoleküle modifiziert vorlagen. Die Konjugate hatten folgende Größen: 50 kDa 10%; 100 kDa 60%; 150 kDa 20%; 200 kDa 5%; 250 kDa 2%. Alle Konjugate wiesen ein Molverhältnis Peroxidase:Polyethylenimin von 1 auf.

Herstellung der Sonde

Zirkulaire, doppelsträngige DNA-Moleküle wurden mit einer Restriktionsendonuklease linearisiert und ohne weitere Reinigung verwendet. 1 µg DNA in 20 µl 5 mM Natriumphosphat pH 6,8 wurde hitzedenaturiert (100°C, 3 Minuten) und 3 Minuten auf Eis abgekült. Zuerst wurden 20 µl Lösung A zugesetzt, danach 6 µl einer 5%igen Glutardialdehydlösung. Die Probe wurde 10 min bei 37°C inkubiert und dann entweder direkt der Hybridisierungsreaktion zugesetzt, welche Nitrocellulosepapier und Hybridisierungslösung enthielt oder durch Zugabe von 28 µl einer Lösung, welche 40% Polyethylenglykol 8000 (Signa) enthielt, gefällt. Die Mischung wurde 6 Minuten zentrifugiert und der Niederschlag in 10 µl, 1,5 M L-Lysin in 5 mM Natriumphosphat, pH 6,8 aufgelöst und für die Hybridisierung eingesetzt. DNA-Bindungsuntersuchungen mit Gelfiltrationssäulen (Sepharose CL-6B) zeigten, daß etwa 25% der Peroxidaseaktivität kovalent an die DNA gebunden war, was einem Protein:DNA Massenverhältnis von etwa 30 entspricht.

Hybridisierung und Visualisierung der Sonden

Nitrocellulosepapier (Schleicher & Schüll) mit immobilisierter DNA wurden eine Stunde bei 38°C eingeweicht in 10×Denhardt's Lösung, 4×SET (1×SET ist 0,15 M NaCl, 0,03 M Tris-HCl, pH 8, 1 mM EDTA) und 0,1% SDS und dann in Kunststoffbehälter überführt, welche zwischen 2 und 20 ml Blank Hybridisierungsmischung enthielten (50% Formamid, 2×Denhardt's Lösung, 4×SET, 0,1%

SDS und 30 µg/ml Hiefe tRNA), unter Schütteln eine Stunde bei 38°C inkubiert und dann nach Zugabe der Sonde 2 bis 16 Stunden bei 38°C weiterinkubiert. Für die Analyse von Hühnchen- und Human-Einfachkopie-Gensequenzen erfolgte die Hybridisierung in Gegenwart von Polyethylenglykol 8000. Die Hybridisierungsmischung wurde modifiziert durch Verwendung von 12% (Gewicht/Volumen) Polyethylenglykollösung in Formamid anstelle von Formamid alleine. Die Nitrocellulosepapier wurden 60 Minuten bei 38°C mit zweimaligem Austausch einer Lösung enthaltend 50% Formamid, 0,4% SDS und 0,5×SSC (1×SSC ist 0,15 M NaCl, 0,015 M Natriumcitrat) und 20 Minuten bei 20°C mit zweimaligem Austausch einer Lösung enthaltend 2×SSC gewaschen. Dann wurden die Filter bei 20°C im Dunklen mit Färbelösung inkubiert. Peroxidase wurde visualisiert mit 10 ml einer Lösung von 100 mM Tris-HCl, pH 7,4, enthaltend 10 mM Imidazol, 2 ml Ethanol, in welchem 6 mg 3,3'-Dianisidin aufgelöst worden waren und 10 µl 30% $H_2O_2$. Nach dem Anfärben wurden die Filter gewaschen und in Tris-HCl-Imidazolpuffer aufgewahrt.

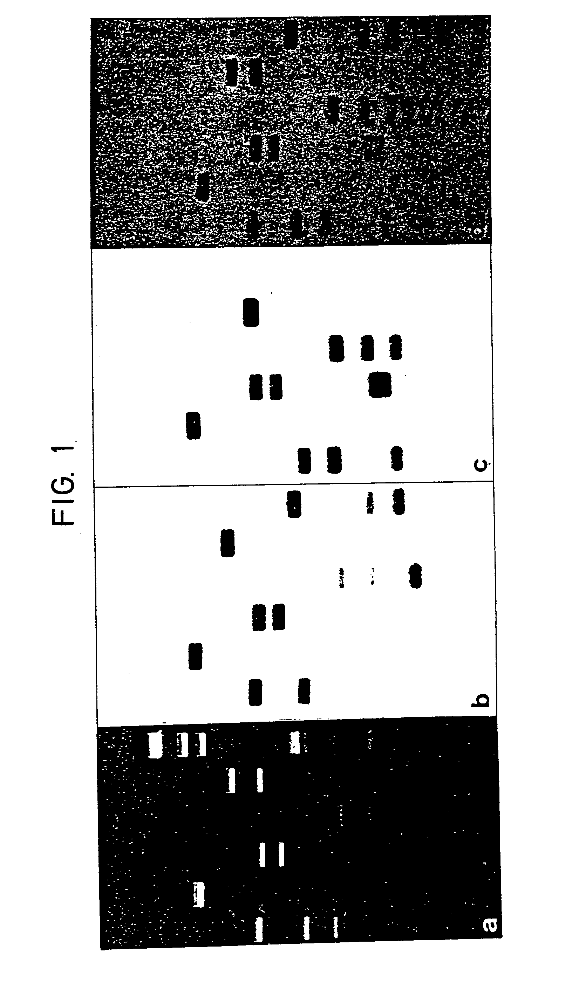

In Fig. 1 zeigt Tafel a ein Agarosegel-Elektrophoretogramm (mit Ethidiumbromid angefärbt) von pHBV 14.1 DNA, die mit verschiedenen Restriktionsendonukleasen gespalten worden war und zwar von links nach rechts mit: BamHI, BglII, AvaI, HpaII, PstI. Jeder Auftrag enthielt 100 ng DNA. Die Spalte ganz rechts enthält Größenmarkierungen folgenden Längen in kb: 23,1, 9,4, 6,6, 4,4, 2,3, 2,2, 2,0, 1,1, 0,75, 0,56, 0,38. (Beispiel 6) Tafel b zeigt ein Nitrocellulosepapier nach Transfer der in Tafel a gezeigten DNA und Hybridisierung mit 0,5 µg von mit EcoRI gespaltener pBR322 DNA, die mit Peroxidase (10 µl von Lösung A) gekuppelt und mit Anisidin-$H_2O_2$ gefärbt wurden. (Beispiel 7).

Tafel c zeigt ein Replikafilter, welches mit HBV DNA-Sequenzen hybridisiert wurde, die mit Peroxidase markiert waren. Das Insert (3,2 kb Länge) von pHBV 14,1 wurde nach Spaltung mit PstI der Agarosegel-Elektrophorese unterzogen und eluiert, 0,5 µg so erhaltener Insert DNA wurde mit Peroxidase markiert (10 µl von Lösung A). (Beispiel 8).

Tafel d zeigt ein Replikafilter, welches mit HBV DNA (mit Peroxidase markiert) und mit pBR322 DNA (mit alkalischer Phosphatase markiert) hybridisiert worden war. 0,5 µg der Insert HBV DNA wurden mit Peroxidase gekuppelt (10 µl Lösung A) und 0,5 µg linearisierte pBR322 DNA wurde mit alkalischer Phosphatase markiert (10 µl Lösung B gemäß Beispiel 5).

Nach der Hybridisierung wurde das Filterpapier zuerst mit der Substratlösung für Phosphatase inkubiert, was in einer blauen Färbung resultierte und dann mit Anisidin für die Sichtbarmachung der Peroxidase (braune Banden). Die Hybridisierungsvolumina und -zeiten betrugen jeweils 15 ml bzw. 3 Stunden. (Beispiel 9).

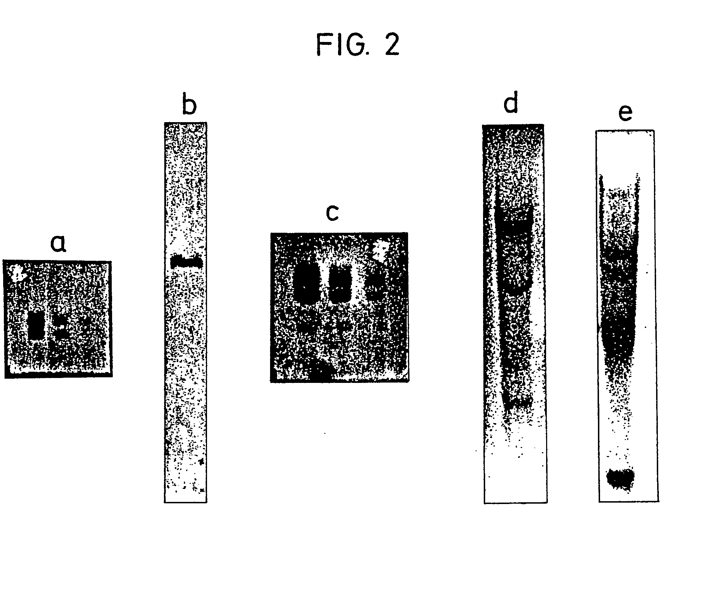

In Fig. 2 zeigt Tafel a das Ergebnis folgenden Versuchs: pDv1, ein pBR322 Plasmid, welches ein Insert von D. virilis enthält, wurde mit EcoRI und PvuII gespalten, wobei vier Fragmente gebildet wurden: 2 D, virilis Fragmente (2,8 und 2,4 kb lang) und 2 pBR322 Fragmente (2,3 und 2,06 kb lang). Serienverdünnungen (500, 100 und 20 pg je Auftrag) wurden elektrophoriert, geblotet und hybridisiert mit 0,5 µg von mit EcoRI linearisiertem pBR322, welches mit Peroxidase (10 µl von Lösung A) 16 Stunden in einem Volumen von 2,0 ml hybridisiert worden war. Die zwei Banden in der mittleren Spur enthalten 24 bzw. 21 pg DNA. Die Fragmente in den beiden Banden sind 2,3 bzw. 2,06 kb lang. (Beispiel 10).

Tafel b) zeigt ein Nitrocellulosepapier nach Transfer von 0,75 µg von mit EcoRI gespaltener une elektrophoretisch aufgetrennter DNA aus D. melanogaster Kc Zellen und dreistündige Hybridisierung in einem Volumen von 5 ml mit 0,5 µg eines mit Pst I linearisierten pBR322 Clons mit einem 4,8 kb Insert, welcher das Alkoholdehydrogenasegen trug und mit Peroxidase (10 µl von Lösung A) gekuppelt war. Die DNA-Fragmente in der Bande sind 4.8 kb lang. (Beispiel 11).

Tafel c) zeigt die Ergenisse eines Titrationsexperiments ähnlich a), hier wurde die Hybridisierung jedoch in Gegenwart von 6% Polyethylenglykol vorgenommen. Die zwei Banden in der ganz rechten Reihe enthalten 4,7 bzw. 4,1 pg DNA. (Beispiel 12).

Tafel d) zeigt die Ergebnisse eines Versuchs, bei dem 10 µg von Hühnerembryo-DNA mit SacI gespalten, der Agarosegel-Elektrophorese unterworfen und dann auf ein Nitrocellulosefilterpapier übertragen worden waren. Die Hybridisierung wurde mit 2,5 µg von mit EcoRI likearisiertem pBR322 Plasmid, enthaltend 2,5 kb codierende Sequenzen für erb A und erb B von Hühnererythroblastosevirus, welches mit Peroxidase (50 µl von Lösung A) gekuppelt worden war, durchgeführt. Die Hybridisierungsdauer betrug 14 Stunden, das Volumen 5 ml. Die Hybridisierungsmischung enthielt 6% Polyethylenglykol. Die Banden entsprechen folgenden Größen in kb: 8,1, 7,5, 5,0, 3,0, 2,5 (Beispiel 13).

Tafel e) zeigt die Ergebnisse eines Versuchs, bei dem 15 µm menschlicher DNA aus HeLa-Zellen mit Hind III gespalten, durch Agarosegel-Elektrophorese der Größe nach aufgetrennt und auf ein Nitrocellulosefilterpapier übertragen und 15 Stunden (in einem Volumen von 5 ml) hybridisiert worden waren unter Verwendung von 2,5 µg eines mit EcoRI linearisierten pKT218-Plasmids, welches die codierenden Regionen des Albumingens (2 kb) enthielt und mit Peroxidase (50 µl) Lösung A) gekuppelt war. Die Hybridisierungsmischung enthielt 6% Polyethylenglykol. Die erhaltenen Banden entsprechenden der folgenden Größen in kb: 6,2, 4,2, 1,8. (Beispiel 14).

In der beigefügten Zeichnung sind die erfindungsgemäß erhaltenen Ergebnisse der Beispiele 6 bis 14 anhand der erhaltenen Elektrophoretogramme näher erläutert. In der Zeichnung stellen dar:

Fig. 1 die Ergebnisse von Blot Hybridisierungsversuchen unter Verwendung erfindungsgemäß markierter Sonden.

Fig. 2 die Ergebnisse von Titrations- und Genom-Blot-Experimenten.

Beispiel 5

Wie im Beispiel 4 beschrieben, wurde ein Konjugat von alkalischer Phosphatase und Polyethylenimid hergestellt. Hierzu wurden 3 mg (350 µl) alkalische Phosphatase aus Kälberdarm (Grad 1, Boehringer Mannheim) gegen 0,1 M Natriumphosphat, pH 6,0 dialysiert. 90 µl der p-Benzochinonlösung, wie im Beispiel 4, wurden zugesetzt und im Dunkeln eine Stunde bei 37°C inkubiert. Nach Gelchromatographie (6 ml Kolonne Sephadex G 25) wurden die Phosphatase enthaltenden Fraktionen vereinigt (etwa 900 µl) und mit 100 µl M NaHCO₃ und 20 µg Polyethylenimin versetzt. Die Mischung wurde 18 Stunden bei 37°C im Dunkeln inkubiert, gegen 5 mM Natriumphosphat pH 6,8 dialysiert und bei 3°C gelagert Lösung B).

Die Herstellung der Sonde erfolgte, wie im Beispiel 4 beschrieben, unter Verwendung von Lösung B anstelle von Lösung A. Auch die Hybridisierung erfolgte wie im Beispiel 4 beschrieben.

Die alkalische Phosphatase wurde visualisiert durch Zusatz von 15 ml einer Lösung, welche Nitroblau-Tetrazolin (NBT) und 5-Brom-4-chlor-3-indoxyl-phosphat-4-toluidin-Salz (BCIP) enthielt und wie folgt hergestellt worden war: 5 mg NBT wurden in 5 ml 0,1 M Tris-HCl pH 9,5, 0,1 M NaCl, 5 mM MgCl₂ (Puffer A) suspendiert, eine Minute heftig gerührt und kurz zentrifugiert. Der Überstand wurde in 10 ml Puffer A abdekantiert, der auf 37°C vorgewärmt worden war. Dann wurden 2,5 mg BCIP in 50 µl Dimethylformamid gelöst und unter Rühren in die NBT-Lösung eingetropft. Nach der Farbentwicklung wurden die Filter gewaschen und in 100 mM Tris-HCl pH 7,4, enthaltend 5 mM EDTA, gelagert.

**Patentansprüche**

1. Verfahren zum Nachweis einer Target-Polynukleotid-Sequenz in Polynukleotidmaterial durch In-Kontakt-Bringen des Materials unter Hybridisierungsbedingungen mit einer Sonde enthaltend einer Polynukleotid Sequenz, die zu der Target-Sequenz komplementär ist und anschließend Verwenden der Sonde zum Nachweis der Target-Sequenz in dem Material, dadurch gekennzeichnet, daß die Sonde durch Reaktion in Lösung der einzelsträngigen komplementären Polynukleotid-Sequenz mit einem nachweisbaren positive geladenen Nukleinsäure bindenden Protein oder Polyamin und durch kovalentes Verbinden des resultierenden Komplexes mit einem Vernetzungsmittel gebildet wurde.

2. Verfahren nach Anspruch 1, worin das Material mit der Sonde in Anwesenheit eines Polyethylenglykols in Kontakt gebracht wird.

3. Verfahren nach Anspruch 2, worin das Polyethylenglykol in einer Konzentration von etwa 6%(Gew/v) anwesend ist.

4. Verfahren nach einem der Ansprüche 1 bis 3, worin die Sonde, die an ein Biotin markiertes Histon H1 Protein kovalent gebundene komplementäre Polynukleotid-Sequenz umfaßt und worin, nach Durchführung der Hybridisierungsreaktion das resultierende hybridisierte Polynukleotid mit einem Avidin-Enzym-Komplex unter solchen Bedinungen in Kontakt gebracht wird, daß das Avidin sich mit dem Biotin der Sonde verbindet und das Enzym anschließend zum Nachweis der Target-Sequenz verwendet wird.

5. Sonde, die zur Verwendung in Hybridisierungsreaktionen geeignet ist, dadurch gekennzeichnet, daß die Sonde durch Reaktion in Lösung einer einzelsträngigen Polynukleotid-Sequenz, die zu einer gewünschten Target-Polynukleotid-Sequenz komplementär ist, mit einem nachweisbaren positiv geladenen Nukleinsäure bindenden Protein oder Polyamin und durch kovalentes Verbinden des resultierenden Komplexes mit einem Vernetzungsmittel gebildet wurde.

6. Sonde nach Anspruch 5, worin das einzelsträngige Polynukleotid kovalent mit dem nachweisbaren positiv geladenen Nukleinsäure bindenden Protein oder Polyamin verbunden ist.

7. Sonde nach Anspruch 5 oder Anspruch 6, worin ein nachweisbares positiv geladenes Nukleinsäure bindendes Protein verwendet wird.

8. Sonde nach Anspruch 7, worin das Protein Histon H1 ist.

9. Sonde nach einem der Ansprüche 5 bis 8, worin das Gewichtsverhältnis von Protein zu Nukleinsäure von 0,1 bis 1,5 ist.

10. Sonde nach einem der Ansprüche 5 bis 9, worin das Protein oder Polyamin markiert ist.

11. Sonde nach Anspruch 10, worin die Markierung ein radioaktives Isotop, eine Farbe, eine Farbverbindung, ein Protein oder ein Enzym ist.

12. Verfahren nach einem der Ansprüche 1 bis 4, worin die verwendete Sonde wie in einem der Ansprüche 5 bis 11 beschrieben, ist.

13. Verfahren zum Nachweis einer Target-Polynukleotid-Sequenz in Polynukleotid-Material durch In-Kontakt-Bringen des Materials unter Hybridisierungsbedingungen mit einer Sonde enthaltend eine Polynukleotid-Sequenz, die zu der Target-Sequenz komplementär ist und durch anschließend Verwenden der Sonde zum Nachweis der Target-Sequenz in dem Material, dadurch gekennzeichnet, daß die Sonde durch Reaktion in Lösung der einzelsträngigen komplementären Polynukleotid-Sequenz mit einem nachweisbaren Protein, das mit einem positiv geladen Protein oder Polyamin modifiziert wurde und durch kovalentes Verbinden des resultierenden Komplexes mit einem Vernetzungsmittel gebildet wurde.

14. Verfahren nach Anspruch 13, worin die Hybridisierungsreaktion in Anwesenheit von Polyethylenglykol durchgeführt wird.

15. Verfahren nach Anspruch 14, worin das Polyethylenglykol in einer Konzentration von etwa 6% (Gew/v) anwesend ist.

16. Sonde, die zur Verwendung in Hybridisierungsreaktionen geeignet ist, dadurch gekennzeichnet, daß die Sonde durch Reaktion in Lösung einer einzelsträngigen Polynukleotid-Sequenz, die zu einer gewünschten Target-Polynukleotid-Sequenz komplementär ist, mit einem nach weisbaren Protein, das mit einem positiv geladenen Protein oder Polyamin modifiziert wurde und durch kovalentes Verbinden des resultierenden Komplexes mit einem Vernetzungsmittel gebildet wurde.

17. Sonde nach Anspruch 16, worin das nachweisebare Protein ein Enzym ist.

18. Sonde nach Anspruch 17, worin das Enzym Meerrettich-Peroxidase oder alkalische Phosphatase ist.

19. Sonde nach einem der Ansprüche 16 bis 18, worin das Polyamin Polyethylenimin ist.

20. Sonde nach Anspruch 19, worin das Gewichtsverhältnis von Protein/Polyethylenimin zu Nukleinsäure von 1:1 bis 30:1 ist.

21. Sonde nach Anspruch 19 oder Anspruch 20, worin das Polyethylenimin ein Molekulargewicht von etwa 1400 hat und im wesentlichen gleiche Mengen primärer, sekundärer und tertiärer Aminogruppen enthält.

22. Verfahren nach einem der Ansprüche 13 bis 15, worin die verwendete Sonde wie in einem der Ansprüche 16 bis 21 beschrieben ist.

23. Reaktionsprodukt eines Enzyms mit einem Polyethylenimin, das mit einem Vernetzungsmittel an eine Polynukleotid-Sequenz kovalent gebunden werden kann.

24. Reaktionsprodukt nach Anspruch 23, worin das Enzym Meerrettich-Peroxidase oder alkalische Phosphatase ist.

25. Reaktionsprodukt nach Anspruch 23 oder Anspruch 24, worin das Polyethylenimin ein Molekulargewicht von etwa 1400 hat und im wesentlichen gleiche Mengen primärer, sekundärer und tertiärer Amingruppen enthält.

26. Verfahren zur Herstellung eines Enzym-Polyamin Konjugates, umfassend das kovalente Anbringen von Benzochinon-Molekülen an ein Enzym und das Reagieren des resultierenden Zwischenstoffes mit einem Polyamin.

27. Verfahren nach Anspruch 26, worin das Polyamin ein Polyethylenimin ist.

28. Verfahren nach Anspruch 27, worin das Polyethylenimin ein Molekulargewicht von etwa 1400 hat und im wesentlichen gleiche Mengen primärer, sekundärer und tertiärer Amingruppen enthält.

## Revendications

1. Procédé pour détecter une séquence polynucléotidique cible dans une matière polynucléotidique, par mise en contact de la matière, dans des conditions d'hybridation, avec une sonde contenant une séquence polynucléotidique complémentaire de la séquence cible, puis utilisation de la sonde pour la détection de la séquence cible dans la matière, procédé caractérisé en ce que la sonde a été formée par réaction, en solution, de la séquence polynucléotidique complémentaire simple brin avec une protéine ou une polyamine décelable, chargée positivement et capable de s'unir à un acide nucléique, et par union covalente du complexe résultant avec un agent de réticulation.

2. Procédé selon la revendication 1 dans lequel la matière est mise en contact avec la sonde en présence d'un polyéthlène-glycol.

3. Procédé selon la revendication 2 dans lequel le polyéthylène-glycol est présent en une concentration d'environ 6% (poids/volume).

4. Procédé selon l'une quelconque des revendications 1 à 3, dans lequel la sonde comprend la séquence polynucléotidique complémentaire liée par covalence à une protéine, l'histone H1, marquée par la biotine, et dans lequel, après avoir effectué la réaction d'hybridization, on met en contact le polynucléotide hybridé obtenu avec un complexe avidine-enzyme dans des conditions telles que l'avidine s'unisse à la biotine de la sonde, et on utilise ensuite l'enzyme pour la détection de la séquence cible.

5. Sonde utilisable dans des réactions d'hybridation, sonde caractérisée en ce qu'elle a été formée par réaction, en solution, d'une séquence polynucléotidique simple brin complémentaire d'une séquence polynucléotidique cible cherchée, avec une protéine ou une polyamine décelable, chargée positivement et s'unissant à un acide nucléique, et par union covalente du complexe formé avec un agent de réticulation.

6. Sonde selon la revendication 5 dans laquelle le polynucléotide simple brin est uni par covalence à la protéine ou à la polyamine décelable, chargée positivement et se liant à un acide nucléique.

7. Sonde selon la revendication 5 ou la revendication 6, pour laquelle on a utilisé une protéine décelable, chargée positivement et se liant à un acide nucléique.

8. Sonde selon la revendication 7 dans laquelle la protéine est l'histone H1.

9. Sonde selon l'une quelconque des revendications 5 à 8, dans laquelle le rapport pondéral de la protéine à l'acide nucléique est compris entre 0,1 et 1,5.

10. Sonde selon l'une quelconque des revendications 5 à 9, dans laquelle la protéine ou la polyamine est marquée.

11. Sonde selon la revendication 10 dans laquelle le marquage est réalisé par un isotope radioactif, une couleur, un composé coloré, une protéine ou une enzyme.

12. Procédé selon l'une quelconque des revendications 1 à 4, dans lequel la sonde utilisée est conforme à l'une quelconque des revendications 5 à 11.

13. Procédé pour déceler une séquence polynucléotidique cible dans une matière polynucléotidique, par mise en contact de la matière, dans des conditions d'hybridation, avec une sonde contenant une séquence polynucléotidique complémentaire de la séquence cible, puis utilisation de la sonde pour la détection de la séquence cible dans la matière, procédé caractérisé en ce que la sonde a été formée par réaction, en solution, de la séquence polynucléotidique complémentaire simple brin avec une protéine décelable qui a été modifiée avec une protéine ou une polyamine chargée positivement, et par union covalente du complexe résultant avec un agent de réticulation.

14. Procédé selon la revendication 13 dans lequel la réaction d'hybridation est effectuée en présence de polyéthylène-glycol.

15. Procédé selon la revendication 14 dans lequel le polyéthylène-glycol est présent en une concentration d'environ 6% (poids/volume).

16. Sonde utilisable pour des réactions d'hybridation, sonde caractérisée en ce qu'elle a été formée par réaction, en solution, d'une séquence polynucléotidique simple brin complémentaire d'une séquence polynucléotidique cible cherchée, avec une protéine décelable qui a été modifiée avec une protéine ou une polyamine chargée positivement, et union covalente du complexe résultant avec un agent de réticulation.

17. Sonde selon la revendication 16 dans laquelle la protéine décelable est une enzyme.

18. Sonde selon la revendication 17 dans laquelle l'enzyme est une peroxydase de raifort ou une phosphatase alcaline.

19. Sonde selon l'une quelconque des revendications 16 à 18, dans laquelle la polyamine est une polyéthylène-imine.

20. Sonde selon la revendication 19 dans laquelle le rapport pondéral entre l'ensemble protéine/polyéthylène-imine et l'acide nucléique est comprise entre 1:1 et 30:1.

21. Sonde selon la revendication 19 ou la revendication 20, dans laquelle la polyéthylène-imine a un poids moléculaire d'environ 1400 et contient des quantités pratiquement égales de radicaux amino primaires, secondaires et tertiaires.

22. Procédé selon l'une quelconque des revendications 13 à 15, dans lequel la sonde utilisée est conforme à l'une des revendications 16 à 21.

23. Produit résultant de la réaction d'une enzyme avec une polyéthylène-imine, produit qui peut être lié par covalence, au moyen d'un réticulant, à une séquence polynucléotidique.

24. Produit réactionnel selon la revendication 23 dans lequel l'enzyme est une peroxydase de raifort ou une phosphatase alcaline.

25. Produit réactionnel selon la revendication 23 ou la revendication 24, dans lequel la polyéthylèneimine a un poids moléculaire d'environ 1400 et contient des quantités pratiquement égales de radicaux amino primaires, secondaires et tertiaires.

26. Procédé pour préparer un conjugué enzyme-polyamine, procédé selon lequel on unit par covalence des molécules de benzoquinone à une enzyme et on fait réagir le corps intermédiaire obtenu avec une polyamine.

27. Procédé selon la revendication 26 dans lequel la polyamine est une polyéthylène-imine.

28. Procédé selon la revendication 27 dans lequel la polyéthylène-imine a un poids moléculaire d'environ 1400 et contient des quantités pratiquement égales de radicaux amino primaires, secondaires et tertiaires.

## Claims

1. A method of detecting a target polynucleotide sequence in polynucleotide material by contacting the material under hybridizing conditions with a probe containing a polynucleotide sequence complementary to the target sequence and thereafter using the probe to detect the target sequence in the material, characterized in that the probe has been formed by reacting in solution the single stranded complementary polynucleotide sequence with a detectable positively charged nucleic acid binding protein or polyamine and covalently binding the resulting complex with a cross-linking agent.

2. A method as claimed in claim 1, wherein the material is contacted with the probe in the presence of a polyethylene glycol.

3. A method as claimed in claim 2, wherein the polyethylene glycol is present in a concentration of about 6% (w/v).

4. A method as claimed in any one of claims 1 to 3, wherein the probe comprises the complementary polynucleotide sequence covalently bound to a biotin-labelled histone H1 protein, and wherein, after the hybridization reaction has been effected, the resulting hybridized polynucleotide is contacted with an avidin-enzyme complex under conditions to cause the avidin to bind to the biotin of the probe, and the enzyme is thereafter used to detect the target sequence.

5. A probe suitable for use in hybridization reactions, characterized in that the probe has been formed by reacting in solution a single stranded polynucleotide sequence, complementary to a desired target polynucleotide sequence, with a detectable positively charged nucleic acid binding protein or polyamine and covalently binding the resulting complex with a cross-linking agent.

6. A probe as claimed in claim 5, wherein the single-stranded polynucleotide is covalently bound to the detectable positively charged nucleic acid binding protein or polyamine.

7. A probe as claimed in claim 5 or claim 6, wherein a detectable positively charged nucleic acid binding protein is used.

8. A probe as claimed in claim 7, wherein the protein is histone H1.

9. A probe as claimed in any one of claims 5 to 8, wherein the weight ratio of protein to nucleic acid is from 0.1 to 1.5.

10. A probe as claimed in any one of claims 5 to 9, wherein the protein or polyamine is labelled.

11. A probe as claimed in claim 10, wherein the label is a radioactive isotope, a dye, a dye compound, a protein or an enzyme.

12. A method as claimed in any one of claims 1 to 4, wherein the probe used is as claimed in any one of the claims 5 to 11.

13. A method of detecting a target polynucleotide sequence in polynucleotide material by contacting the material under hybridizing conditions with a probe containing a polynucleotide sequence complementary to the target sequence and thereafter using the probe to detect the target sequence in the material, characterized in that the probe has been formed by reacting in solution the single stranded complementary polynucleotide sequence with a detectable protein which has been modified with a positively charged protein or polyamine, and covalently binding the resulting complex with a cross-linking agent.

14. A method as claimed in claim 13, wherein the hybridization reaction is performed in the presence of polyethylene glycol.

15. A method as claimed in claim 14, wherein the polyethylene glycol is present in a concentration of about 6% (w/v).

16. A probe suitable for use in hybridization reactions, characterized in that the probe has been formed by reacting in solution a single stranded polynucleotide sequence, complementary to a desired target polynucleotide sequence, with a detectable protein which has been modified with a positively charged protein or polyamine, and covalently binding the resulting complex with a cross-linking agent.

17. A probe as claimed in claim 16, wherein the detectable protein is an enzyme.

18. A probe as claimed in claim 17, wherein the enzyme is horseradish peroxidase or alkaline phosphatase.

19. A probe as claimed in any one of claims 16 to 18, wherein the polyamine is polyethyleneimine.

20. A probe as claimed in claim 19, wherein the weight ratio of protein/polyethyleneimine to nucleic acid is from 1:1 to 30:1.

21. A probe as claimed in claim 19 or claim 20, wherein the polyethyleneimine has a molecular weight of about 1400 and contains substantially equal amounts of primary, secondary and tertiary amino groups.

22. A method as claimed in any one of claims 13 to 15, wherein the probe used is as claimed in any one of claims 16 to 21.

23. The reaction product of an enzyme with a polyethyleneimine which reaction product has the ability to be covalently bound with a cross-linking agent to a polynucleotide sequence.

24. The reaction product claimed in claim 23, wherein the enzyme is horseradish peroxidase or alkaline phosphatase.

25. The reaction product of claim 23 or claim 24, wherein the polyethyleneimine has a molecular weight of about 1400 and contains substantially equal amounts of primary, secondary and tertiary amine groups.

26. A method of making an enzyme-polyamine conjugate, which method comprises covalently attaching benzoquinone molecules to an enzyme and reacting the resulting intermediate with a polyamine.

27. A method as claimed in claim 26, wherein the polyamine is a polyethyleneimine.

28. A method as claimed in claim 27, wherein the polyethyleneimine has a molecular weight of about 1400 and contains substantially equal amounts of primary, secondary and tertiary amine groups.

FIG. 1

# FIG. 2